(19) Europäisches Patentamt
European Patent Office
Office européen des brevets

(11) Publication number: 0 004 433 B1

# (12) EUROPEAN PATENT SPECIFICATION

(45) Date of publication of patent specification: 18.05.83

(21) Application number: 79300388.0

(22) Date of filing: 13.03.79

(51) Int. Cl.[3]: C 07 D 213/64, A 01 N 43/40 //C07D213/73, C07D213/61

## (54) Herbicidal 2-(p-substituted-phenoxy)-pyridines and process for preparing them.

(30) Priority: 17.03.78 GB 1074278

(43) Date of publication of application: 03.10.79 Bulletin 79/20

(45) Publication of the grant of the patent: 18.05.83 Bulletin 83/20

(84) Designated Contracting States: BE CH DE FR GB IT NL

(56) References cited:
EP - A - 0 000 483
DE - A - 1 793 212
FR - A - 2 329 632
FR - A - 2 347 349

(73) Proprietor: IMPERIAL CHEMICAL INDUSTRIES PLC
Imperial Chemical House Millbank
London SW1P 3JF (GB)

(72) Inventor: Cartwright, David
1 Stonehaven Drive Woodley
Reading Berkshire (GB)
Inventor: Hunt, John Desmond
24 Goodwood Close
Midhurst Sussex (GB)

(74) Representative: Fawcett, Richard Fennelly et al,
Imperial Chemical Industries PLC Legal Department: Patents Thames House North Millbank
London SW1P 4QG (GB)

EP 0 004 433 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Herbicidal 2-(p-substituted-phenoxy) pyridines and process for preparing them

This invention relates to certain pyridine compounds and to a process for preparing them. The compounds have herbicidal activity and are also useful as intermediates for preparing other herbicides.

According to the present invention there is provided a compound of formula (I):

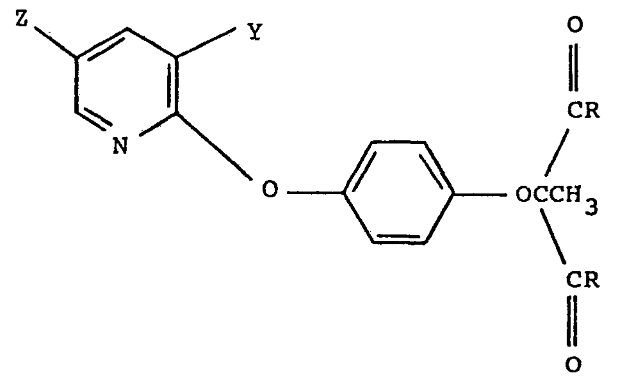

(I)

wherein Z is trifluoromethyl, Y is hydrogen or chlorine, and each of the groups R, which may be the same or different, is hydroxy, $C_{1-4}$ alkoxy, a group of formula —OM wherein M is a cation, or optionally alkyl-, phenyl- or pyrid-2-yl-substituted amino.

Particular examples of compounds according to the invention are the compounds of formula II below:

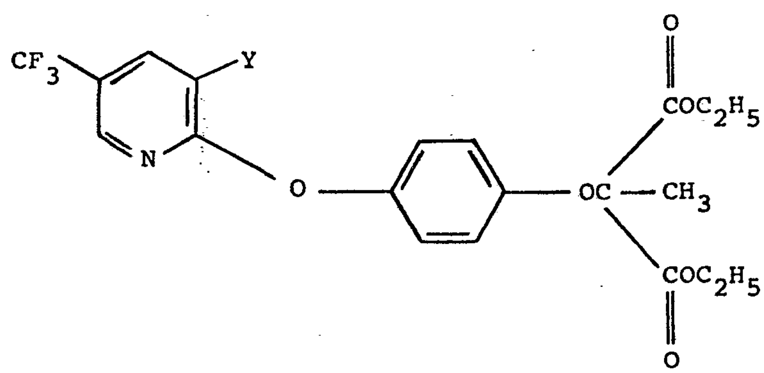

(II)

wherein Y is hydrogen or chlorine.

The compounds of the invention are herbicides which are in general substantially more effective against grass species than against broad-leaved species of plant. They may be used to control unwanted grass species growing alone, or at suitable rates of application they may be used to control grass weeds growing among broad-leaved crop plants. The compounds may be either applied to the soil before the emergence of the unwanted grass species (pre-emergence application) or to the above-ground parts of growing grass plants (post-emergence application).

The compounds can be used for inhibiting the growth of unwanted plants, particularly grass species, by applying to the plants, or to the locus thereof, a herbicidally effective amount of a compound of formula (I) as hereinbefore defined.

The amount of the compound to be applied will depend upon a number of factors, for example the particular plant species whose growth is to be inhibited, but in general an amount of from 0.1 to 5 kilograms per hectare is usually suitable. The skilled worker in the art will readily be able to determine suitable amounts for use by means of standardised routine tests, without undue experimentation.

The compounds of the invention are preferably applied in the form of compositions, in which the active ingredient is mixed with a carrier comprising a solid or liquid diluent. Preferably the composition further comprises a surface-active agent.

The solid compositions of the invention may be for example, in the form of dusting powders, or may take the form of granules. Suitable solid diluents include, for example, kaolin, bentonite, kieselguhr, dolomite, calcium carbonate, talc, powdered magnesia, and Fuller's earth.

Solid compositions may also be in the form of dispersible powders or grains comprising in addition to the active ingredient, a wetting agent to facilitate the dispersion of the powder or grains in liquids. Such powders or grains may include fillers, suspending agents and the like.

Liquid compositions include aqueous solutions, dispersions and emulsions containing the active ingredient preferably in the presence of one or more surface-active agents. Water or organic liquids

may be used to prepare solutions, dispersions, or emulsions of the active ingredient. The liquid compositions of the invention may also contain one or more corrosion inhibitors for example lauryl isoquinolinium bromide.

Surface active agents may be of the cationic, anionic or non-ionic type. Suitable agents of the cationic type include for example quaternary ammonium compounds, for example cetyltrimethyl ammonium bromide. Suitable agents of the anionic type include for example soaps, salts of aliphatic mono-esters of sulphuric acid, for example sodium lauryl sulphate; and salts of sulphonated aromatic compounds, for example dodecylbenzene-sulphonate, sodium, calcium and ammonium lignosulphonate, butylnaphthalene sulphonate, and a mixture of the sodium salts of diisopropyl- and triisopropyl-naphthalenesulphonic acid. Suitable agents of the non-ionic type include, for example, the condensation products of ethylene oxide with fatty alcohols such as oleyl alcohol and cetyl alcohol, or with alkyl phenols such as octylphenol, nonylphenol, and octylcresol. Other non-ionic agents are the partial esters derived from long chain fatty acids and hexitol anhydrides, for example sorbitol monolaurate; the condensation products of the said partial esters with ethylene oxide and the lecithins.

The compositions which are to be used in the form of aqueous solutions, dispersions or emulsions are generally supplied in the form of a concentrate containing a high proportion of the active ingredient, the concentrate being diluted with water before use. These concentrates are usually required to withstand storage for prolonged periods and after such storage to be capable of dilution with water in order to form aqueous preparations which remain homogeneous for a sufficient time to enable them to be applied by conventional spray equipment. In general concentrates may conveniently contain from 10 to 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

The compounds of the invention may be prepared for example by the route outlined in Scheme A below:—

Scheme A

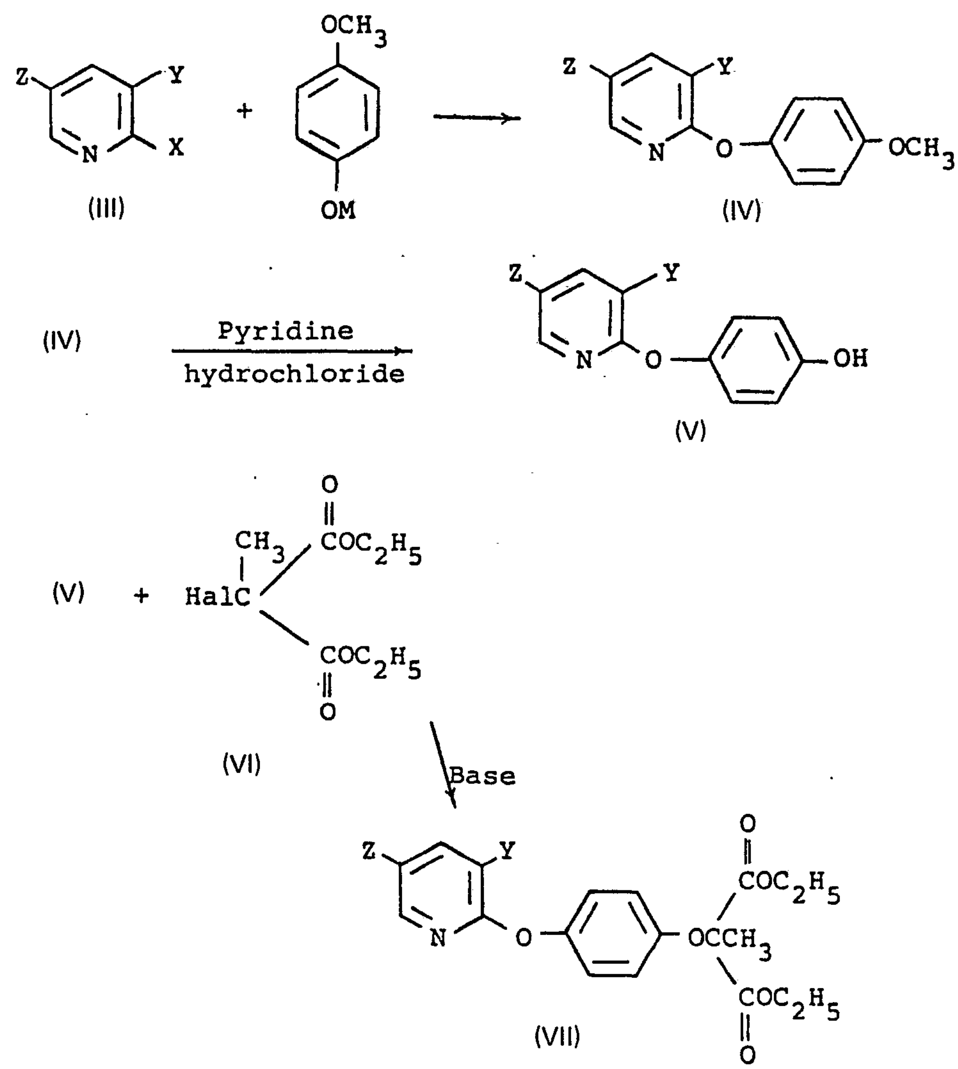


(III) (IV)

(IV) (V)

(V) (VI)

(VII)

In Scheme A, the symbols Z and Y have the meanings previously assigned to them, X and Hal stand for a halogen, preferably chlorine or bromine, and M is a cation, for example sodium.

In Scheme A, a suitably substituted halogeno-pyridine (III) is reacted with a metal salt of p-methoxy-phenol, for example the sodium salt of p-methoxyphenol. The reaction is preferably carried out in a solvent or diluent, for example methyl ethyl ketone, tetrahydrofuran; dimethylsulphoxide or dimethylacetamide. The p-methoxy-phenoxy compound (IV) so obtained is then demethylated by a standard procedure, for example by heating with pyridine hydrochloride or with hydrogen bromide in acetic acid, to obtain the corresponding p-hydroxy compound (V). This in turn is reacted in the presence of a base (for example an alkali metal carbonate, for example potassium carbonate) with the appropriate halogeno-malonic acid derivative (VI) to obtain the required compound (formula I, $R=OC_2H_5$). Preferably this reaction is carried out in a solvent or diluent, for example methyl ethyl ketone.

The product of the reaction sequence shown in Scheme A is a diester. Compounds according to the invention wherein the group R is other than an alkoxy group may be prepared from compounds in which R is an alkoxy group by conventional chemical procedures.

Thus, compounds in which the group R is an OM group may be prepared by alkaline hydrolysis of compounds in which R is an alkoxy group.

Thus compounds in which —OM represents an —ONa group may be prepared by hydrolysis of compounds in which R is alkoxy with aqueous sodium hydroxide, and isolating the sodium salt by a conventional procedure. Compounds wherein R is an amino or substituted amino group may be prepared by treating compounds in which R is an alkoxy group with ammonia or an appropriately substituted amine, according to known procedures.

In an alternative process for preparing the compounds of the invention, a 4-hydroxyphenoxy substituted malonate ester of formula (VIII) may be reacted in the form of its metal salt with a halogenopyridine (III) as shown in Scheme B.

Scheme B

(III) +

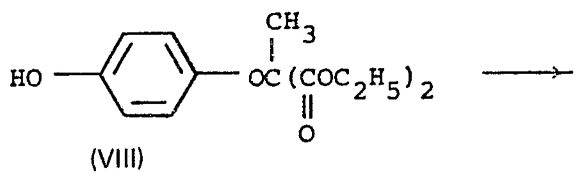

(VII)

(VIII)

The reaction is illustrated in Scheme B using a diethyl malonate ester, but the free acid or esters other than ethyl may be used if desired. Generally lower alkyl esters (i.e. esters in which the alkyl group has 1 to 6 carbon atoms) are preferred. The product (VII) may, as noted for Scheme A, be transformed into other compounds of the invention by conventional methods if so desired. The metal salt of the hydroxy compound (VIII) in Scheme B may be for example an alkali metal, for example ltihium, sodium or potassium. The metal salt may be preformed by reacting compound (VIII) with, for example, sodium hydride or solid sodium or potassium hydroxide, preferably while the hydroxy compound (VIII) is in solution. Examples of solvents include aprotic solvents, for example dimethylformamide dimethylsulphoxide, and tetramethylenesulphone. The metal salt having been prepared, the halogenopyridine (III) may then be added. The reaction with the halogenopyridine may be accelerated by heating, for example to a temperature of 70 to 125°C for a period of from 1 to 3 hours at atmospheric pressure. The product (VII) may be isolated by conventional methods, for example by evaporating the solvent under reduced pressure, diluting the residue with water and extracting with a water-immiscible organic solvent, for example chloroform. The chloroform extract is dried and evaporated to give the product which may be further purified by conventional methods if desired.

In an alternative method of carrying out the reaction of Scheme B, the hydroxy compound (VIII) and the halogenopyridine (III) are heated together in an aprotic solvent, for example methyl ethyl ketone in the presence of a molar proportion of a base, for example an alkali metal carbonate, for example anhydrous sodium or potassium carbonate, and the product (VII) isolated by conventional methods. When the solvent is methyl ethyl ketone, the reaction is conveniently carried out at the reflux temperature of this solvent.

The hydroxy compounds (VIII) required as starting materials for Scheme B may be prepared by the reaction shown in Scheme C.

Scheme C

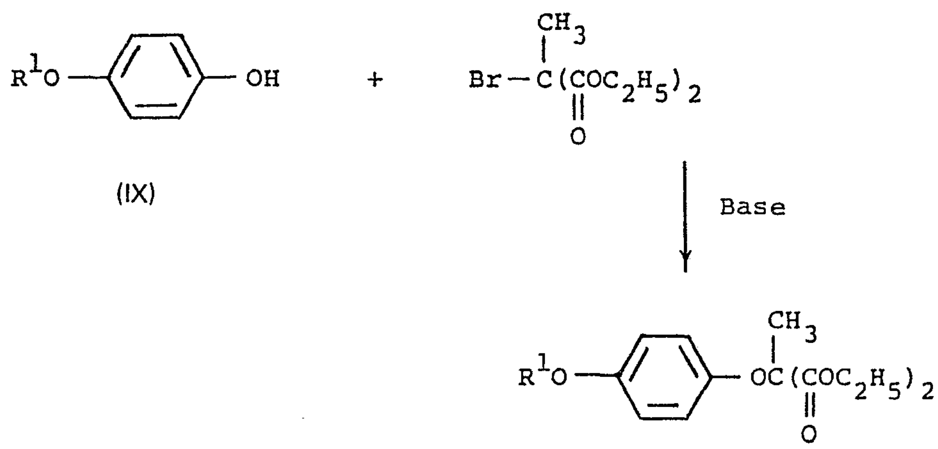


(IX)

(X)

In Scheme C, the symbol $R^1$ stands for hydrogen or a protecting group for example a lower alkyl group or benzyl group. The reaction is preferably carried out in a solvent, for example a lower alkanol or a mixture of a lower alkanol and water. The base may be, for example, an alkali metal hydroxide or carbonate. When $R^1$ is hydrogen, the required hydroxy compound (VIII) may be obtained directly by the process of Scheme C. Where $R^1$ is a lower alkyl or benzyl protecting group, a further reaction step is necessary to remove this group and produce the hydroxy compound (VIII). When $R^1$ is a lower alkyl group, conventional methods of removing such a group include heating the compounds (X) with pyridine hydrochloride or with concentrated hydrobromic acid. When $R^1$ is a benzyl group it may be removed as toluene by hydrogenation of the compound (X) in the presence of a platinum or other noble metal catalyst.

The starting materials (III) used in the process of Scheme A may be prepared by halogenation of appropriate 2-halogeno-5- or 3-methylpyridines. Thus, 2-chloro-5-trifluoromethylpyridine may be obtained by chlorinating 2-bromo-5-methylpyridine in the presence of ultra-violet light to obtain 2-chloro-5-trichloromethyl-pyridine. The 2-chloro-5-trifluoromethylpyridine is obtainable by reacting the 2-chloro-5-trichloromethylpyridine with a fluorinating agent, for example antimony trifluoride or liquid hydrogen fluoride.

In an alternative process, 2-halogeno-3- or 5-trifluoromethyl pyridines may be obtained by reacting a 2-halogeno-3- or -5-carboxypyridine with sulphur tetrafluoride in the presence of hydrogen fluoride.

The compounds of the invention, apart from their use as herbicides, are also useful as intermediates for the preparation of a further class of herbicidal compounds. In another aspect, therefore, the compounds can be used for preparing herbicidal compounds of the formula (XI)

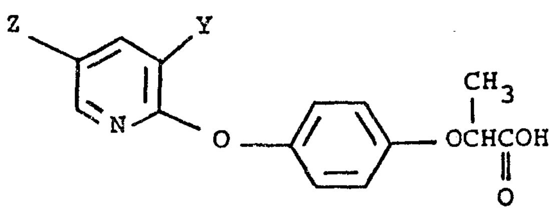

(XI)

wherein Z and Y are as defined above, by a process which comprises heating a compound of the formula (I) as hereinbefore defined wherein each group R is an —OH group to a temperature at which loss of carbon dioxide takes place and recovering the compound of formula (XI). The temperature at which loss of carbon dioxide ("decarboxylation") takes place may vary somewhat from one compound to another but may readily be ascertained by simply heating a small sample of the compound and observing the temperature at which gas evolution becomes evident. In general, decarboxylation will take place at a conveniently rapid rate at temperatures from 100° to 175°C. The reaction may if desired be carried out in a diluent or solvent. In some cases water may be a convenient solvent and in others a higher boiling solvent may be preferred, for example toluene, xylene, or dimethylformamide. The product may be recovered be conventional methods, for example by evaporation of the solvent under reduced pressure. The herbicidal properties of the compounds (XI) have been described, together

with those of related compounds, in published UK Patent Specification No 2002368. If desired, the compounds (XI) may be converted into their corresponding salts, esters, amides, and nitriles by conventional methods, and these derivatives may also be used as herbicides. Salts may be prepared by neutralisation of the acids (XI) with basis; for example sodium and potassium salts may be prepared by neutralisation with a molar proportion of sodium or potassium hydroxide in aqueous solution. Esters may be prepared by reaction with alkanols according to conventional procedures. Particular examples of esters include the butyl ester of the compound (XI) in which Z is $CF_3$ and Y is hydrogen, and the propyl ester of the compound (XI) in which Z is $CF_3$ and Y is chlorine.

As noted above, Schemes A and B for preparing compounds of formula (I) give rise to ester derivatives of formula (VII) whereas the free acids (formula I, R=OH) are required as starting materials for the decarboxylation reaction described above for preparing compounds of formula (XI). The free acids (formula I, R=OH) may be obtained for example by alkaline hydrolysis of compounds of formula I wherein each R is an alkoxy group, under mild conditions. Thus for example a compound of formula I wherein each R is ethyl may be dissolved in aqueous isopropanol or ethanol and treated with a molar proportion of dilute sodium hydroxide at room temperature. When hydrolysis is complete the solution is made acid with, for example, hydrochloric acid and the compound of formula I wherein each R is OH may be extracted with an organic solvent, for example chloroform, and used as a starting material for the decarboxylation process described above.

The invention is illustrated by the following Examples, in which all parts are by weight and all temperatures in degrees Centigrade unless otherwise specified.

## Example 1

This Example illustrates the preparation of a compound according to the invention, namely, the compound of formula (II) hereinabove wherein Y is chlorine.

(a) *Preparation of 2-amino-3-bromo-5-methylpyridine*

2-Amino-5-methylpyridine (108 g) in glacial acetic acid (300 ml) was heated to 90—100°C while bromine (160 g) in acetic acid (55 ml) was slowly added with stirring. When addition was complete, the mixture was stirred and heated for a further 30 minutes and then allowed to cool overnight. The solid which separated was filtered off and mixed with ice and the mixture neutralised with concentrated ammonia, keeping the temperature at 0 to 5°C. The solid was collected, washed with water, and dried to give the bromo-compound.

(b) *Preparation of 3-bromo-2-chloro-5-methylpyridine*

The product from (a) (145 g) was dissolved in concentrated hydrochloric acid (750 ml) and water (450 ml) and the solution cooled to −10°C. Sodium nitrite (54 g) in cold water (450 ml) was added dropwise with stirring over a period of 90 minutes while the mixture was kept at −5°C. The solution was stirred for a further 2 hours, and then basified with concentrated ammonia, keeping the temperature below 20°C. The solid which separated was washed with water, dried, dissolved in ether (1500 ml) and washed with cold sodium hydroxide solution (1M; 1 litre). The ether solution was washed twice with water (1 litre portions), dried, and evaporated to give the required 3-bromo-2-chloro-5-methylpyridine.

(c) *Preparation of 2,3-dichloro-5-trichloromethyl pyridine*

The product from (b) (64 g) in dry carbon tetrachloride (650 ml) was treated with dry hydrogen chloride. The precipitate was broken up and the suspension heated under reflux while dry chlorine was bubbled into the mixture, with illumination from an ultra-violet light source. After 4½ hours, the mixture was cooled, filtered, and the filtrate evaporated to give the required 2,3-dichloro-5-trichloromethyl-pyridine. The mass spectrum was consistent with the structure assigned to this compound.

(d) *Preparation of 2,3-dichloro-5-trifluoromethyl pyridine*

The product from (c) (1.0 g) and antimony trifluoride (3.0 g) were heated together at 170—180° for 30 minutes. The mixture was then cooled, mixed with ice and water, and extracted with ether. The ether extracts gave a brown oil containing a mixture of 2,3-dichloro-5-trifluoromethylpyridine and 3-chloro-2-fluoro-5-trifluoromethylpyridine with a minor amount of 2,3-dichloro-3-chlorodifluoro-methylpyridine.

(e) *Preparation of 3-chloro-2-p-methoxyphenoxy-5-trifluoromethylpyridine*

*p*-Methoxyphenol (1.5 g) was added to a suspension of sodium hydride (0.6 g 50% oil dispersion, washed with petroleum) in dry dimethyl sulphoxide (30 ml) and the mixture stirred for 15 minutes. A solution of the combined products (1.5 g) from several preparations carried out as described in paragraph (d), in dimethylsulphoxide (20 ml) was added to the reaction mixture and heated to 60°C for four hours. A further amount of sodium hydride (0.3 g of 50% oil dispersion, washed with petroleum), and potassium carbonate (1.38 g) was added. Heating was continued for another 4 hours. The mixture

was poured into ice and water, and extracted with ether (400 ml). The ether extracts were washed with water, dilute sodium hydroxide, and water, dried, and evaporated to give the product.

(f) *Preparation of 3-chloro-2-p-hydroxyphenoxy-5-trifluoromethylpyridine*

The product from (e) (2 g) was heated with pyridine hydrochloride (20 g) at 170—180°C for 6 hours. The mixture was cooled, diluted with dilute hydrochloric acid, and extracted with ether. The ether extracts gave an oily solid which was purified by preparative thin layer chromatography using silica as the adsorbent and 6% ethanol-chloroform as the solvent.

(g) *Preparation of compound of formula II wherein Y=chlorine*

The product from (f) (0.64 g) was dissolved in methyl ethyl ketone and heated under reflux with diethyl alpha bromo alpha methyl malonate (0.56 g) and anhydrous potassium carbonate (0.73 g) for 3 hours and then left to stand overnight at room temperature. The mixture was filtered and the filtrate was evaporated and the residue purified by preparative scale thin layer chromatography, using silica gel as the solid phase and a mixture of 20 volumes of ether with 100 volumes of hexane as the solvent. The chromatogram developed a single band which on elution gave a clear oil (0.84 g) identified as compound II (Y=Cl) by its nuclear magnetic resonance spectrum.

Example 2

Following the procedure of Example 1, but using 2-chloro-5-trifluoromethylpyridine as starting material, in place of 2,3-dichloro-5-trifluoromethyl pyridine, the compound of formula II wherein Y is hydrogen was prepared. The 2-chloro-5-trifluoromethyl pyridine required as starting material was prepared as follows:

6-Chloronicotinic acid (23.6 g), sulphur tetrafluoride (37.4 g) and anhydrous hydrogen fluoride (18.7 g) were heated in an autoclave with stirring for 8 hours at 120°. The mixture was cooled, poured on to ice, and neutralised with concentrated sodium hydroxide at 0°. The mixture was extracted with ether and the extracts washed with water, dried, and evaporated. The residue was distilled and the fraction boiling at 140—150° collected. Analysis indicated that this consisted of 2-chloro-5-trifluoromethylpyridine with some 2-fluoro-5-trifluoromethylpyridine.

Example 3

This Example illustrates the herbicidal properties of the compound according to the invention of formula I wherein Z is $CF_3$, Y is hydrogen, and R is ethoxy. The compound was formulated for test by mixing an appropriate amount with 5 ml of an emulsion prepared by diluting 160 ml of a solution containing 21.8 grams per litre of Span 80 and 78.2 grams per litre of Tween 20 in methyl cyclohexanone to 500 ml with water. Span 80 is a Trade Mark for a surface-active agent comprising sorbitan monolaurate. Tween 20 is a Trade Mark for a surface-active agent comprising a condensate of twenty molar proportions of ethylene oxide with sorbitan mono-oleate. The mixture of the compound and the emulsion was shaken with glass beads and diluted to 40 ml with water.

Seeds of the test plants listed in the following Table were placed on the surface of fibre trays of soil and were sprayed with the composition at the rate of 1000 litres per hectare. This gave an application rate of 0.2 kilograms of compound per hectare. The seeds were then covered with a thin layer of soil. Three weeks after spraying, the seedlings in the sprayed fibre trays were compared with the seedlings in unsprayed control trays, the damage being assessed on a scale of 0 to 5 where 0 is 0 to 20% damage and 5 is complete kill. The results are given in the Table below:—

| TEST PLANTS | | | | | | | | | | | | | | | | | | | | | | | |
|---|---|---|---|---|---|---|---|---|---|---|---|---|---|---|---|---|---|---|---|---|---|---|---|
| Sb | Rp | Ct | Sy | Mz | Ww | Rc | Sn | IP | Am | Pi | Ca | Po | Xs | Ab | Cv | Ot | Dg | Pu | St | Ec | Sh | Ag | Cn |
| 0 | 0 | 1 | 0 | 2 | 0 | 0 | 1 | 3 | 0 | 0 | 0 | 0 | 0 | 2 | – | 0 | 2 | 0 | 0 | 0 | 2 | 0 | 0 |

*Names of test plants*

| | |
|---|---|
| Sb | Sugar beet |
| Rp | Rape |
| Ct | Cotton |
| Sy | Soya bean |
| Mz | Maize |
| Ww | Winter wheat |
| Rc | Rice |
| Sn | *Senecio vulgaris* |
| Ip | *Ipomoea purpurea* |
| Am | *Amaranthus retroflexus* |
| Pi | *Polygonum aviculare* |
| Ca | *Chenopodium album* |
| Po | *Portulaca oleracea* |
| Ab | *Abutilon theophrastii* |
| Xs | *Xanthium spinosa* |
| Cv | *Convolvulus arvensis* |
| Ot | Cultivated oats |
| Dg | *Digitaria sanguinalis* |
| Pu | *Poa annua* |
| St | *Setaria viridis* |
| Ec | *Echinochloa crus-galli* |
| Sh | *Sorghum halepense* |
| Ag | *Agropyron repens* |
| Cp | *Cyperus rotundus* |

**Claims**

1. A compound of formula (I):—

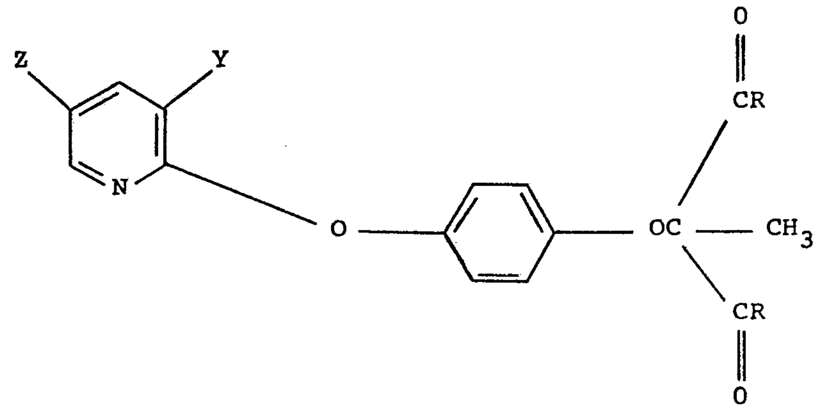

wherein Z is trifluoromethyl, Y is hydrogen or chlorine, and each of the groups R, which may be the same or different, is hydroxy, $C_{1-4}$ alkoxy, a group of formula —OM wherein M is a cation, or optionally alkyl-, phenyl- or pyrid-2-yl-substituted amino.

2. A process of preparing a compound according to Claim 1, characterised by reacting in the presence of a base a compound of formula:

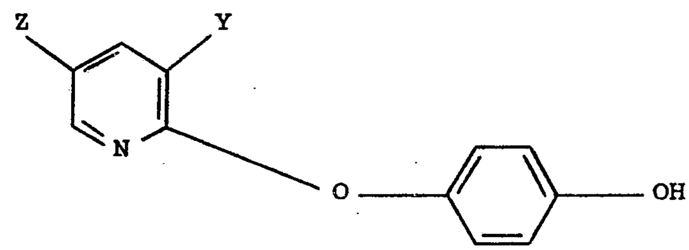

wherein Z and Y are as defined in Claim 1, with a compound of formula:

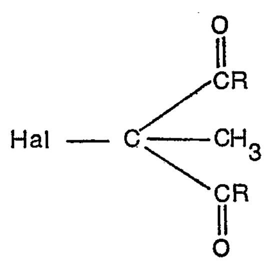

wherein each of the groups R, which may be the same or different, is $C_{1-4}$ alkoxy, and Hal is halogen, to give a compound of general formula (I) wherein both groups R are $C_{1-4}$ alkoxy, and, when the groups R are not the required groups, forming said groups by known methods.

**Patentansprüche**

1. Verbindung der Formel (I):

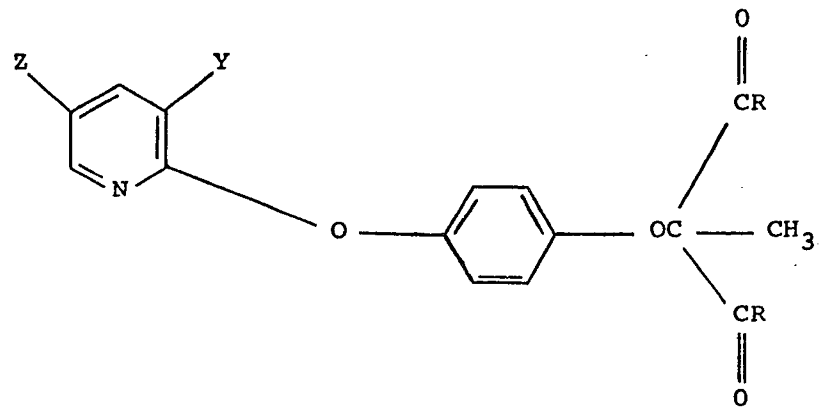

worin Z für Trifluoromethyl steht, Y für Wasserstoff oder Chlor steht, und jede der Gruppen R, welche gleich oder verschieden sein können, für Hydroxy, $C_{1-4}$-Alkoxy, eine Gruppe der Formel —OM, wobei M ein Kation bedeutet, oder ggf. alkyl-, phenyl- oder pyrid-2-yl-substituiertes Amino steht.

2. Verfahren zur Herstellung einer Verbindung nach Anspruch 1, dadurch gekennzeichnet, daß man in Gegenwart einer Base eine Verbindung der Formel:

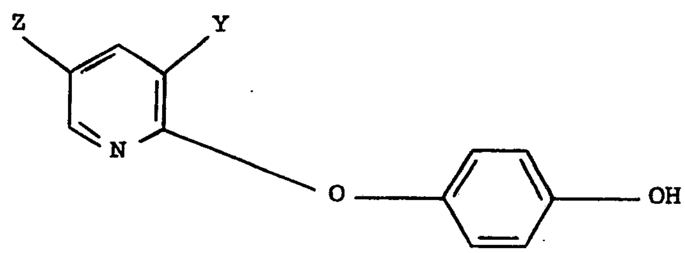

worin Z und Y die in Anspruch 1 angegebene Definition besitzen, mit einer Verbindung der Formel:

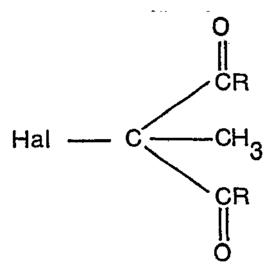

worin jede der Gruppen R, welche gleich oder verschieden sein können, für $C_{1-4}$-Alkoxy steht und Hal für Halogen steht, unter Bildung einer Verbindung der allgemeinen Formel (I), worin beide Gruppen R für $C_{1-4}$-Alkoxy stehen, umsetzt und, wenn die Gruppen R nicht die gewünschten Gruppen sind, diese Gruppen durch bekannte Verfahren bildet.

**Revendications**

1. Un composé de formule (I):

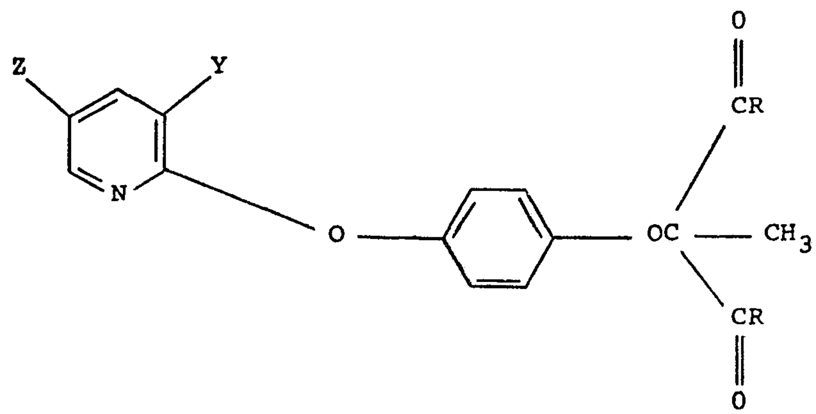

dans laquelle Z est le groupe trifluorométhyle, Y est l'hydrogène ou le chlore et chacun des groupes R, qui peuvent être égaux ou différents, est un radical hydroxy, alkoxy en $C_1$ à $C_4$, un groupe de formule —OM dans laquelle M est un cation, ou à titre de variante, un groupe amino à substituant alkyle, phényle ou pyrid-2-yle.

2. Procédé de préparation d'un composé suivant la revendication 1, caractérisé par la réaction, en présence d'une base, d'un composé de formule:

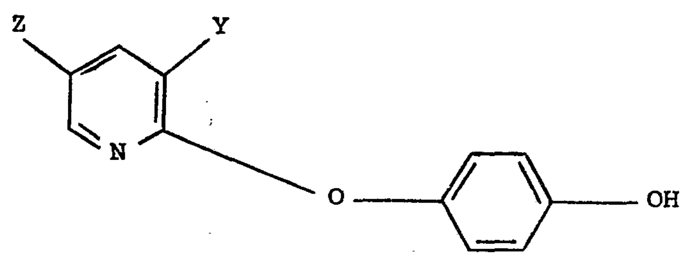

dans laquelle Z et Y ont la définition donnée dans la revendication 1, avec un composé de formule:

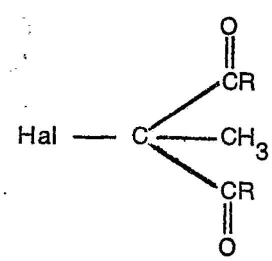

chacun des groupes R, qui peuvent être égaux ou différents étant un groupe alkoxy en $C_1$ à $C_4$ et Hal étant un halogène, pour former un composé de formule générale (I) dans laquelle les deux groupes R sont des groupes alkoxy en $C_1$ à $C_4$, et, lorsque les groupes R ne sont pas les groupes désirés, formation desdits groupes par des procédés connus.